# EUROPÄISCHE PATENTANMELDUNG

(11) **EP 3 181 150 A1**
(43) Veröffentlichungstag der Anmeldung: **21.06.2017**
(21) Anmeldenummer: 15201444.5
(22) Anmeldetag: 19.12.2015
(51) Int. Cl.: A61K 45/06, A61K 31/167, A61K 31/19, A61K 31/192, A61P 29/00, A61K 9/00

(54) **PHARMAZEUTISCHE ZUBEREITUNGEN**

(71) Anmelder: Analyticon Discovery GmbH, 14473 Potsdam (DE); B.R.A.I.N. Biotechnology Research And Information Network AG, 64673 Zwingenberg (DE)
(72) Erfinder: Siems, Karsten, 14552 Michendorf (DE); Riedel, Katja, 64625 Bensheim (DE); Krohn, Michael, 64653 Lorsch (DE)
(74) Vertreter: Fabry, Bernd

(57) **Zusammenfassung**

Vorgeschlagen wird eine Zubereitung, enthaltend
(a) mindestens einen NSAID-Wirkstoff und
(b) Dehydroabietinsäure oder einen Extrakt, der Dehydroabietinsäure enthält als Arzneimittel.

## Beschreibung

### GEBIET DER ERFINDUNG

Die Erfindung befindet sich auf dem Gebiet der Pharmazie und betrifft Zubereitungen mit NSAID-Wirkstoffen und speziellen Terpenderivaten, die sich durch verbesserte orale Verabreichung und höhere Verträglichkeit auszeichnen.

### STAND DER TECHNIK

Unter dem Kürzel NSAID - auch als NSAR (nichtsteroidale Antirheumatika) - wird eine Gruppe von nicht-opioiden Analgetika zusammengefasst, die ihrer entzündungshemmenden (antiphlogistischen) Wirkung symptombezogen auch zur Rheumatherapie eingesetzt werden. Im Gegensatz dazu werden Glucocorticoide als *steroidale Antirheumatika* bezeichnet. Die Kategorie wurde in den späten 1950er Jahren eingeführt, um die Differenz gegen 'steroidale Antirheumatika' mit ihren teils schweren Nebenwirkungen zu markieren.

NSAID stellen heute die wichtigste Gruppe der Schmerzmittel dar, zumal davon auch so bekannte Stoffe wie Acetylsalicylsäure und Ibuprofen umfasst werden. Gemeinsam ist diesen Stoffen nicht nur ein gleicher Wirkmechanismus, sondern auch der Umstand, dass die orale Aufnahme durch den bitteren, teilweise adstringierenden und metallischen Geschmack erschwert wird und auch durch Süßstoffe nicht oder allenfalls kaum überdeckt werden kann. In vielen Fällen wird die geschmackliche Beeinträchtigung durch Süßstoffe wie beispielsweise Acesulfam K oder Saccharin sogar noch verstärkt.

Ein weiteres Problem, das mit der oralen Aufnahme der NSAID-Wirkstoffe verbunden ist, besteht in ihrer unzureichenden Schleimhautverträglichkeit, die bei oraler Verabreichung in der Mundhöhle, speziell im Hals und auf der Zunge zu Brennen und Reizungen führt und ein trocknes Gefühl hervorruft. Diese Beschwerden halten mitunter noch eine ganze Weile an. Ebenfalls kann es zu Beeinträchtigungen der Magenschleimhaut kommen.

Die Aufgabe der vorliegenden Erfindung hat daher darin bestanden, den nachteiligen Eigenschaften der NSAID-Wirkstoffen abzuhelfen und sie in solcher Weise unter Verwendung von geeigneten Hilfsstoffen zu formulieren, dass flüssige oder feste Arzneimittel erhalten werden, die geschmacklich einwandfrei sind und gleichzeitig auch eine verbesserte Schleimhautverträglichkeit aufweisen.

### BESCHREIBUNG DER ERFINDUNG

Ein erster Gegenstand der Erfindung betrifft daher eine Zubereitung, enthaltend
(a) mindestens einen NSAID-Wirkstoff und
(b) Dehydroabietinsäure oder einen Extrakt, der Dehydroabietinsäure enthält, als Arzneimittel.
Ein zweiter Gegenstand ist auf eine Zubereitung gerichtet, enthaltend
(a) mindestens einen NSAID-Wirkstoff und
(b) Dehydroabietinsäure oder einen Extrakt, der Dehydroabietinsäure enthält,
   zur Verwendung als Arzneimittel zur Behandlung und/oder Prophylaxe von Schmerzzuständen, Entzündungszuständen und rheumatischen Erkrankungen.

Überraschenderweise wurde gefunden, dass die Formulierung von NSAID-Wirkstoffen mit Dehydroabietinsäure bzw. Extrakten, die Dehydroabietinsäure (vorzugsweise in Mengen von wenigstens 20 Gew.-%, insbesondere wenigstens 30 Gew.-% und insbesondere 40 bis 60 Gew.-%) die obige komplexe Problemstellung in vollem Umfang löst. Dehydroabietinsäure ist nicht nur in der Lage, den bitteren, adstringierenden und metallischen Geschmack der NSAID zu maskieren, sondern auch die geschmackliche Wahrnehmung von Formulierungen, die NSAID zusammen mit Süßstoffen enthalten, merklich zu verbessern. In vielen Fällen kommt es dabei sogar zu einer angenehmen Verstärkung der Aromen und der Süße der Formulierung.

Ein weiterer wichtiger Aspekt ist darin zu sehen, dass die Dehydroabietinsäure auch die Schleimhautverträglichkeit signifikant verbessert. Entsprechende Formulierungen verursachen entweder gar kein oder nur noch sehr geringes Brennen bei der oralen Aufnahme, auch die Mundfeuchtigkeit wird nicht länger beeinträchtigt. Insgesamt wird die Einnahme von NSAID-Dehydroabietinsäure Arzneimittel von Patienten auch im Hinblick auf die Magenschleimhautverträglichkeit als wesentlich angenehmer empfunden.

### NSAID WIRKSTOFFE (Non-Steroidal Anti-Inflammatory Drugs)

Zu den wichtigsten nichtspezifischen NSAID, die erfindungsgemäß die Gruppe (a) bilden, zählen die so genannten COX-1/2-Hemmer, nämlich:
Gruppe 1: Acetylsalicylsäurederivate (1) 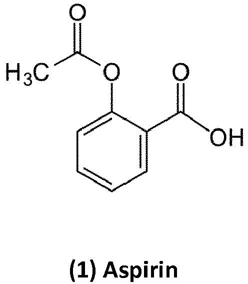
Gruppe 2: Arylpropionsäurederivate (2) bis (6) 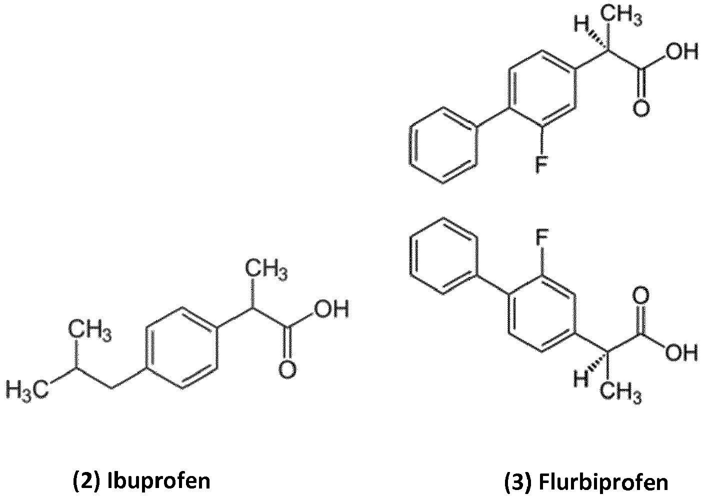 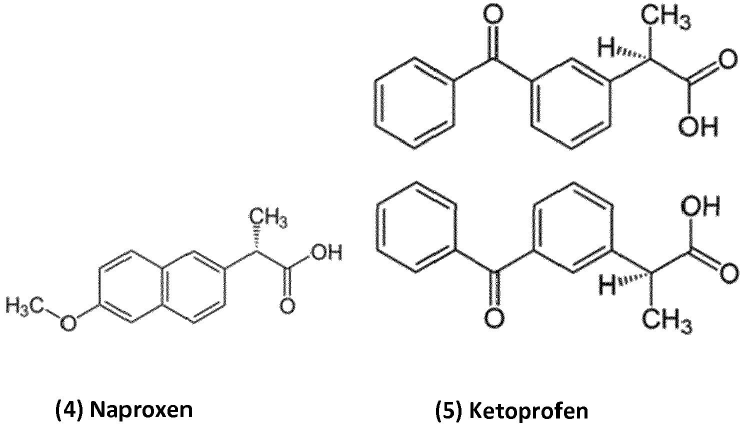 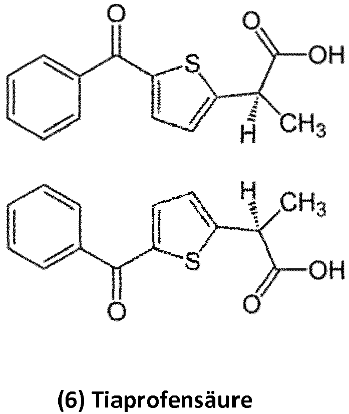
Gruppe 3: Arylessipsäurederivate (7) 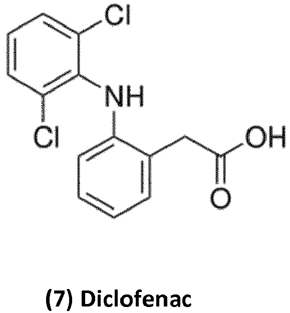
Gruppe 4: Indolessigsäurederivate (8) 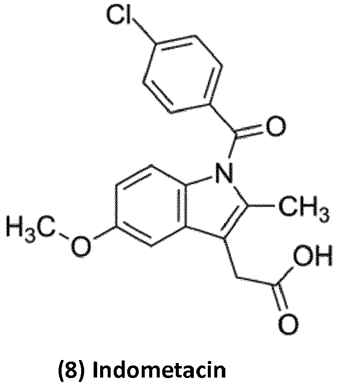
Gruppe 5: Anthranilsäurederivate (9) - (10) 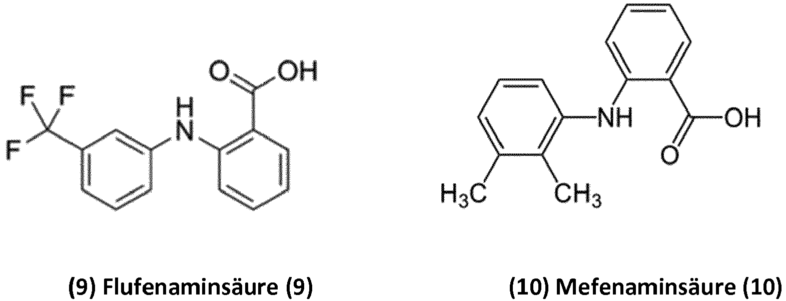
Gruppe 6: Oxicame (11) - (13) 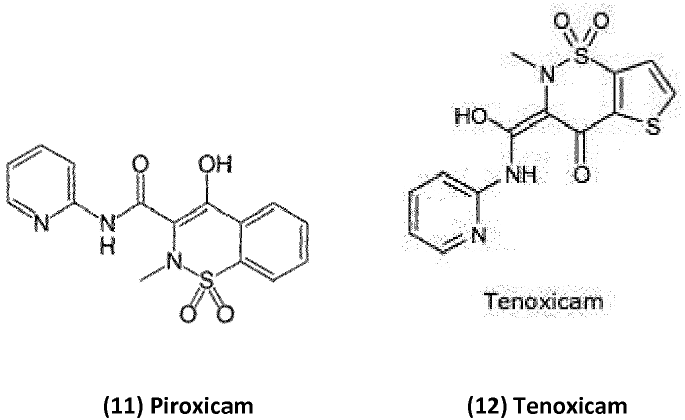 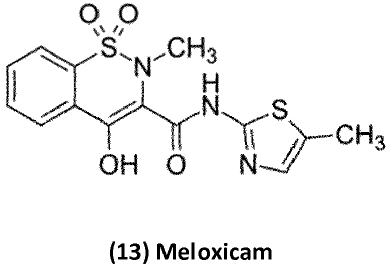
Gruppe 7: Aminophenole

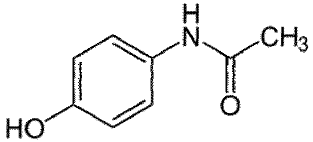

### (14) para Acetylaminophenol (Paracetamol)

### DEHYDROABIETINSÄURE

Das Diterpen Dehydroabietinsäure (Komponente (b)) kommt in vielen Nadelbaumgewächsen (u.a. in den Pflanzenfamilien der Pinaceae und Juniperaceae, dabei insbes. in den Gattungen Pinus, Abies, Larix, Juniperus) sowohl in den Nadeln, als auch in Rinde, Wurzel und insbesondere im Harz vor. Es fällt als Abfallprodukt bei der Papierherstellung an und ist im sog. Tallöl enthalten.

Auch aus anderen Pflanzenfamilien wurde Dihydroabietinsäure isoliert (z.B. aus den Gattungen Illicium, Liquidambar, Styrax, Callicarpa, Rosmarinus, Salvia, Commiphora, Boswellia). Besonders häufig kommt Dehydroabietinsäure in Harzen vor (z.B. Liquidambar, Styrax, Boswellia, Commiphora, Colophonium, aber auch in Bernstein).

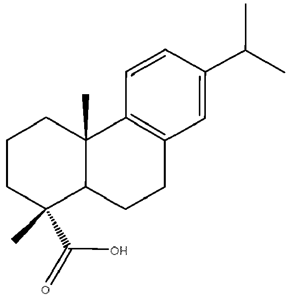

### Strukturformel von Dehydroabietinsäure (CAS 1740-19-8)

Der Gehalt an Dehydroabietinsäure im Kiefernharz beträgt meist unter 10%, kann aber durch eine dem Fachmann als Disproportionierung bekannte Methode (z.B. nach dem von Song et al in JOURNAL OF WOOD CHEMISTRY AND TECHNOLOGY, 5(4), 535-542 (1985) beschriebenen Verfahren) auf einen Gehalt von >50% gesteigert werden.

Es sind beide Enantiomere der Dehydroabietinsäure bekannt (CAS 1740-19-8 und CAS 6980-63-8). Im Sinne der vorliegenden Erfindung sind unter dem Begriff "Dehydroabietin säure" beide Isomere sowie beliebige Enantiomerengemische zu verstehen.

Obschon auch Extrakte, die Dehydroabietinsäure enthalten, und die handelsübliche technische Dehydroabietinsäure mit einem Reinheitsgrad von etwa 85 % eingesetzt werden kann, ist der Einsatz von hochreinen Produkten mit Dehydroabietinsäuregehalten von mindestens 90 Gew.-%, vorzugsweise mindestens Gew.-95 % und insbesondere etwa 95 bis etwa 99 Gew.-% bevorzugt. Solche hochreinen Produkte sind mit den üblichen Aufarbeitungsverfahren der präparativen organischen Chemie erhältlich, so dass der Fachmann hierzu nicht erfinderisch tätig werden muss. Nachfolgend wird im Beispiel 1 ein entsprechendes Verfahren exemplarisch beschrieben.

Anstelle der Dehydroabietinsäure selbst können auch deren Salze, speziell deren Alkali- und Ammoniumsalze, insbesondere das Natriumsalz eingesetzt werden.

Die Erfindung umfasst auch den Einsatz von Dehydroabietinsäure-haltigen Extrakten, deren Herstellung ebenfalls zum Handwerkszeug des Fachmanns gehört und exemplarisch im nachfolgenden Beispiel 2 beschrieben ist. Vorzugsweise enthalten diese Extrakte mindestens 20 Gew.-%, insbesondere mindestens 30 Gew.-% und besonders bevorzugt etwa 40 bis etwa 60 Gew.-% Dehydroabietinsäure.

In Summe bevorzugt ist die Verbesserung des sensorischen Profils von Ibuprofen.

### SÜSSSTOFFE

Als Süßstoffe oder süß schmeckende Zusatzstoffe, welche die fakultative Gruppe (c) bilden, kommen zunächst Kohlenhydrate wie z.B. Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin in Betracht. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch synthetische, d.h. in der Regel enzymatisch hergestellte Stärke oder Zuckerhydrolysate (Invertzucker, Fructosesirup) sowie natürliche oder synthetische süß schmeckende Substanzen, wie
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-Tryptophan, L-Prolin);
- Weitere süß schmeckende niedermolekulare Substanzen, wie z.B. Hernandulcin, Phyllodulcin, Dihydrochalconglykoside, Glycyrrhizin, Glycyrrhizinsäure sowie ihre Derivate und Salze, Rubusoside, Mogroside
- Extrakte süß schmeckender Pflanzen wie Stevia rebaudiana, Glycyrrhiza ssp. (Lakritz), Lippia dulcis, Momordica grosvenori.

Bei den Süßstoffen kann es sich auch um pflanzliche Extrakte handeln, wie nachstehend exemplarisch beschrieben.

**Rebaudioside** gehören zu den Steviosiden, den Hauptbestandteilen der Pflanze *Stevia rebaudiana,* die auch als Süßkraut oder Honigkraut bezeichnet wird.

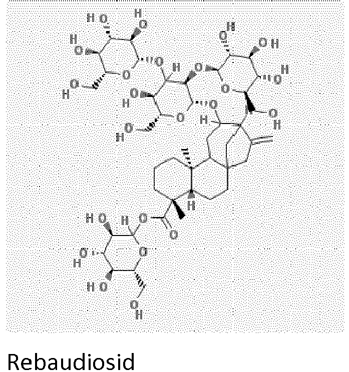

10 % der Trockenmasse der Blätter macht das Diterpenglykoid Steviosid aus, gefolgt von Rebaudiosid A (2 bis 4 Gew.-%) sowie mehr als zehn weiteren Steviolglycosiden, wie etwa dem Dulcosid. Rebaudioside und Stevia-Extrakt sind als Süßungsmitteln inzwischen in den meisten Staaten zugelassen; eine tägliche Aufnahmemenge von bis zu 4 mg Steviosid pro Kilogramm Körpergewicht wird als unbedenklich angesehen. Im Sinne der Erfindung können einzelne Rebaudioside oder die Extrakte der Stevia-Pflanze eingesetzt werden. Besonders bevorzugt ist jedoch die Verwendung von Rebaudiosid A, da dieser Stoff eine geringere Bitterkeit und die höchste Süßkraft aufweist. Die erfindungsgemäßen Stoffgemische können die Komponenten (a) und (b) im Gewichtsverhältnis von etwa 1:99 bis etwa 99:1, vorzugsweise etwa 25:75 bis etwa 75:25 und insbesondere etwa 40:60 bis etwa 60:40 enthalten

Auch die **Dihydrochalcone** stellen Flavonoide dar, wobei insbesondere die beiden Vertreter Naringenin-Dihydrochalcon und Neohesperidin-Dihydrochalcon hervorzuheben sind, die als künstliche Süßungsmittel bekannt sind:

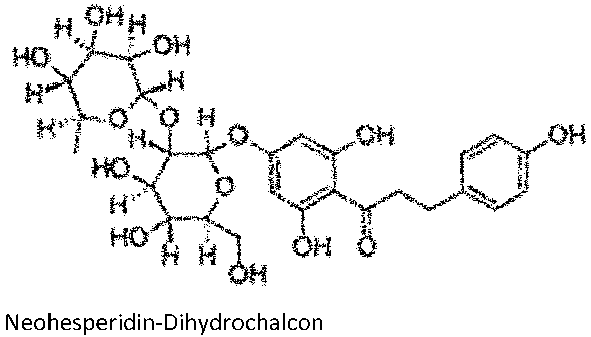

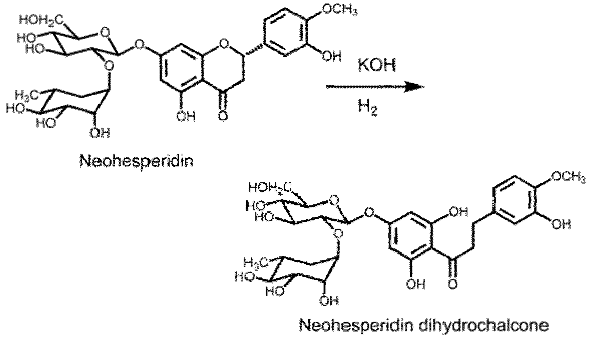

Unter der Bezeichnung Mogroside wird eine Gruppe von Glykosiden des Cucurbitans verstanden, die als Bestandteil des natürlichen Süßungsmittels Luo Han Guo bekannt sind. Hervorzuheben ist hier das Mogrosid-V, das 400mal süßer als Zucker ist.

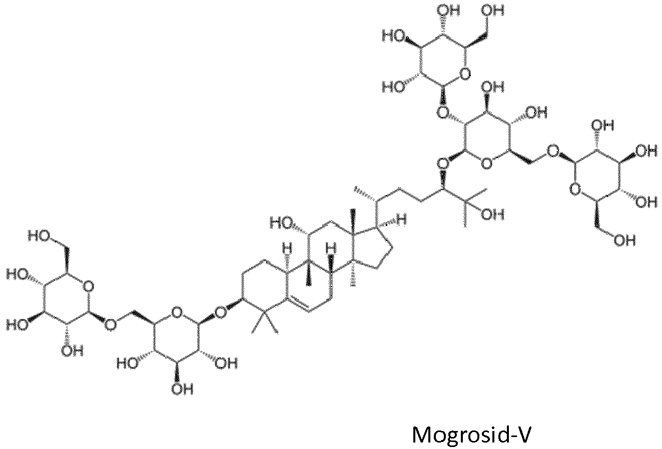

Schließlich kommen als Süßstoffe auch Extrakte der Pflanzen in Betracht, die ausgewählt sind aus der Gruppe, die gebildet wird von *Rubus allegheniensis, Rubus arcticu, Rubus strigosus, Rubus armeniacus, Rubus caesius, Rubus chamaemorus, Rubus corylifolius agg., Rubus fruticosus agg., Rubus geoides, Rubus glaucus, Rubus gunnianus, Rubus idaeus, Rubus illecebrosus, Rubus laciniatus, Rubus leucodermis, Rubus loganobaccus Rubus loxensis, Rubus nepalensis, Rubus nessensis, Rubus nivalis, Rubus odoratus, Rubus pentalobus, Rubus phoenicolasius, Rubus saxatilis, Rubus setchuenensis, Rubus spectabilis* und *Rubus ulmifolius* sowie deren Gemischen. Hierbei handelt es sich im Wesentlichen um Extrakte von unterschiedlichen Brombeer- und Himbeerarten, die einen Gehalt an Rubosiden aufweisen. Bevorzugt sind Extrakte von *Rubus suavissimus.*

Ein weiterer Wirkstoff in dieser Gruppe ist die Glycyrrhizinsäure oder ein entsprechendes Salz oder einen Extrakt, der diesen Stoff enthält.

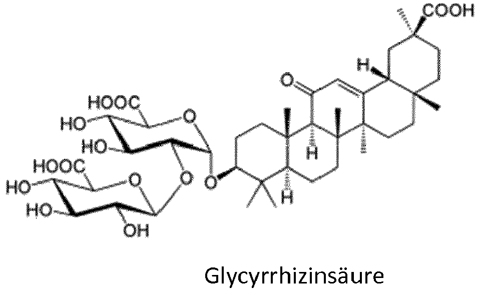

Im Sinne der Erfindung ist es möglich, die Säure selbst, ihre Salze - beispielsweise Natrium-, Kalium- oder Ammoniumsalz - oder die Extrakte der Pflanze *Glycyrrhiza glabra* einzusetzen. Besonders bevorzugt ist das Monoammoniumglycyrrhizat.

Die bevorzugten Süßstoffe, deren geschmackliche Wahrnehmung in der Formulierung mit NSAID-Wirkstoffen es zu verbessern gilt, sind ausgewählt sind aus der Gruppe, die gebildet wird von Saccharin, Acesulfam K, Steviolglykosiden, insbesondere Rebaudiosid A, sowie Stevia-Extrakten

### TRÄGER

In einer bevorzugten Ausführungsform werden die beiden Komponenten (a) und (b) sowie gegebenenfalls (c) nicht alleine eingesetzt, sondern unter Zuhilfenahme eines Trägers formuliert. Die Natur des Trägers ist an sich nicht kritisch, solange dieser nicht über einen unangenehmen Eigengeschmack verfügt. Der Träger kann beispielsweise erforderlich sein, um die Mischung zu tablettieren. Es kann sich aber auch um einen reinen Füllstoff oder ein Verdickungsmittel handelt. Vorzugsweise kommen für diesen Zweck Kohlenhydrate und insbesondere Polysaccharide in Betracht. Hierzu zählen beispielsweise Dextrine, Cellulosen, insbesondere Mikrocellulosen, Stärken und modifizierte Stärken sowie hochpolymere Stoffe wie etwa Xanthan Gum.

Handelt es sich um flüssige Zubereitungen kommen als Träger neben Wasser und Ethanol, toxikologisch unbedenkliche Polyole wie etwa Glycerin oder Ethylenglykol in Betracht.

### ZUBEREITUNGEN

Bei den erfindungsgemäßen Zubereitungen handelt es sich üblicherweise um Arzneimittel. Ebenfalls umfasst werden aber Formulierungen, die rezeptfrei sind und beispielsweise zur Prophylaxe deinen. Insgesamt können die Zubereitungen als feste oder flüssige Formulierungen vorliegen. Beispiele für feste Zubereitungen sind etwa Tabletten, Lutschbonbons, Kaugummis oder Kapseln. Beispiele für flüssige Formulierungen sind etwa Säfte oder Sprays.

Die Zubereitungen weisen vorzugsweise die folgende Zusammensetzung auf:
(a) etwa 0,01 bis etwa 90 Gew.-%, vorzugsweise etwa 0,1 bis etwa 80 Gew.-% und insbesondere etwa 1 bis etwa 50 Gew.-% NSAID-Wirkstoffe;
(b) etwa 0,001 bis etwa 5 Gew.-%, vorzugsweise etwa 0,005 bis etwa 2 Gew.-% und insbesondere etwa 0,01 bis etwa 1 Gew.-% Dehydroabietinsäure oder einen entsprechenden Extrakt,
(c) 0 bis etwa 15, vorzugsweise etwa 1 bis etwa 8 Gew.-% und insbesondere etwa 2 bis etwa 5 Gew.-% Gew.-% Süßstoffe
(d) 0 bis etwa 95 Gew.-%, vorzugsweise 10 bis etwa 90 Gew.-% und insbesondere etwa 25 bis etwa 60 Gew.-% Träger,
wobei als Maßgabe gilt, dass sich die Mengenangaben gegebenenfalls mit weiteren Hilfs- und Zusatzstoffen zu 100 Gew.-% ergänzen.

Zubereitungen mit Polyolen (z.B. Sorbitol) oder Zuckern als Süßstoff enthalten üblicherweise zwischen 5 und 20 Gew.-% Süßstoff, Zubereitungen mit so genannten "High-Intensity-Sweetenern (HIS)" (z.B. Saccharin oder Cyclamat) hingegen weniger als 1 Gew.-% Süßstoff. Häufig werden Mischungen unterschiedlicher Süßstoffe eingesetzt, um den störenden, häufig metallischen oder bitteren Bei- und Nachgeschmack zu minimieren.

Als weitere Hilfs- und Zusatzstoffe kommen insbesondere Farbstoffe und Aromen in Betracht.

### Farbstoffe

Lebensmittelfarbstoffe oder kurz Farbstoffe sind Lebensmittelzusatzstoffe zum Färben von Zubereitungen, die zum Verzehr geeignet sind. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentische Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### Aromastoffe

Die erfindungsgemäßen Zubereitungen können einen oder mehrere Aromastoffe enthalten. Typische Beispiele umfassen: Acetophenon, Allylcapronat, alpha-lonon, betalonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-l-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Im Folgenden wird eine Reihe von galenischen Formen, in denen die erfindungsgemäßen Zubereitungen vorliegen können, näher erläutert.

### TABLETTEN

Tabletten, welche die erfindungsgemäßen Zubereitungen enthalten oder daraus bestehen, können die für diese Darreichungsform weiteren üblichen Bestandteile enthalten, als da beispielsweise sind: Trägerstoffe (wie schon oben erläutert), Sprengmittel und/oder Farb- und Aromastoffe. Die Tablettierung selbst stellt ein hinlänglich bekanntes großtechnisches Verfahren dar. Exemplarisch sei auf die DE 10 2006 051529 A1 (HENKEL) verwiesen, in der die Herstellung von Tabletten ausführlich beschrieben wird.

### Trägerstoffe

Geeignete Trägerstoffe lassen sich in Füll- und Bindemittel untergliedern. Füllmittel sorgen dafür, dass die Tablette die notwendige Größe/Masse erhält. Eingesetzt werden Stärken (Mais-, Kartoffel- und Weizenstärke) und Lactose. Weitere Füllmittel sind: Glucose, Mannitol, Sorbitol. Fructose wird auf Grund ihres hohen Preises nur sehr selten verwendet. Saccharose wird vor allem für Lutschtabletten verwendet.

Bindemittel sorgen hingegen für den Zusammenhalt in den Granulaten oder Pulvern und neben dem Pressdruck für die Festigkeit von Tabletten. Sie unterteilen sich in Trockenbindemittel wie z. B. MCC (mikrokristalline Cellulose) oder Stärke und in Feuchtbindemittel/Klebstoffe für die Granulierung wie z. B. Stärkekleister, Celluloseether, Kollidon und Gelatine.

### Sprengmittel

Um den Zerfall vorgefertigter Formkörper zu erleichtern, ist es möglich, Desintegrationshilfsmittel, sogenannte Tablettensprengmittel, in diese Mittel einzuarbeiten, um die Zerfallszeiten zu verkürzen. Unter Tablettensprengmitteln bzw. Zerfallsbeschleunigern werden Hilfsstoffe verstanden, die für den raschen Zerfall von Tabletten in Wasser oder anderen Medien und für die zügige Freisetzung der Wirkstoffe sorgen.

Diese Stoffe, die auch aufgrund ihrer Wirkung als "Spreng" mittelbezeichnet werden, vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen lässt. Altbekannte Desintegrationshilfsmittel sind beispielsweise Carbonat/Zitronensäure-Systeme, wobei auch andere organische Säuren eingesetzt werden können. Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden Desintegrationsmittel auf Cellulosebasis eingesetzt. Reine Cellulose weist die formale Bruttozusammensetzung (C₆H₁₀O₅)ₙ auf und stellt formal betrachtet ein beta-1,4-Polyacetat von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymer analoge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht allein als Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.- percent, besonders bevorzugt unterhalb 20Gew.- percent, bezogen auf das Desintegrationsmittel auf Cellulosebasis. Besonders bevorzugt wird als Desintegrationsmittel auf Cellulosebasis reine Cellulose eingesetzt, dief rei von Cellulosederivaten ist.

Die als Sprengmittel eingesetzte Cellulose wird vorzugsweise nicht in feinteiliger Form eingesetzt, sondern vor dem Zumischen zu den zu verpressenden Vorgemischen in eine gröbere Form überführt, beispielsweise granuliert oder kompaktiert. Die Teilchengrößen solcher Desintegrationsmittel liegen zumeist oberhalb 200 µm vorzugsweise zu mindestens 90 Gew.-% zwischen 300 und 1.600 µm und insbesondere zu mindestens 90 Gew.-% zwischen 400 und 1.200 µm.

Als weiteres Sprengmittel auf Cellulosebasis oder als Bestandteil dieser Komponente kann mikrokristalline Cellulose eingesetzt werden. Diese mikrokristalline Cellulose wird durch partielle Hydrolyse von Cellulosen unter solchen Bedingungenerhalten, die nur die amorphen Bereiche (ca. 30 % der Gesamt-Cellulosemasse) der Cellulosen angreifen und vollständig auflösen, die kristallinen Bereiche (ca. 70 %) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind.

Erfindungsgemäß bevorzugt können darüber hinaus weiterhin gasentwickelnde Brausesysteme eingesetzt werden. Das gasentwickelnde Brausesystem kann aus einer einzigen Substanzbestehen, die bei Kontakt mit Wasser ein Gas freisetzt. Unter diesen Verbindungen ist insbesondere das Magnesiumperoxid zu nennen, das bei Kontakt mit Wasser Sauerstofffreisetzt. Üblicherweise besteht das gasfreisetzende Sprudelsystem jedoch seinerseits aus mindestens zwei Bestandteilen, die miteinander unter Gasbildung reagieren. Während hier eine Vielzahl von Systemen denk- und ausführbar ist, die beispielsweise Stickstoff, Sauerstoff oder Wasserstoff freisetzen, wird sich das in den Wasch-und Reinigungsmittel eingesetzte Sprudelsystem sowohl anhand ökonomischer als auch anhand ökologischer Gesichtspunkte auswählen lassen. Bevorzugte Brausesysteme bestehen aus Alkalimetallcarbonat und/oder - hydrogencarbonat sowie einem Acidifizierungsmittel, das geeignet ist, aus den Alkalimetallsalzen in wässriger Lösung Kohlendioxidfreizusetzen.

### KAUGUMMIS

Bei den erfindungsgemäßen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird. Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

### KAPSELN

Unter Kapseln, die die erfindungsgemäßen Zubereitungen enthalten können, sind sphärische Aggregate zu verstehen, die mindestens einen festen oder flüssigen Kern enthalten, der von mindestens einer kontinuierlichen Hülle umschlossen ist. Die Wirkstoffe können durch Überzugsmaterialien verkapselt werden und dabei als Makrokapseln mit Durchmessern von etwa 0,1 bis etwa 5 mm oder Mikrokapseln mit Durchmessern von etwa 0,0001 bis etwa 0,1 mm vorliegen.

### Überzugsmaterialien

Geeignete Überzugsmaterialien sind dabei beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nicht-modifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Unter diesen Stoffen ist Gelatine besonders bevorzugt, da sie gut verfügbar ist und mit unterschiedlichen Schwellfaktoren bezogen werden kann.

Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Ebenfalls bevorzugt sind Alginatkapseln wie sie beispielsweise in den folgenden Schriften ausführlich beschrieben werden: EP 0389700 A1, US 4,251,195, US 6,214,376, WO 2003 055587 oder WO 2004 050069 A1.

In einer weiteren bevorzugten Ausführungsform besteht die Hülle der Kapseln aus Melamin-Formaldehydharzen oder Koazervationsprodukten aus kationischen Monomeren oder Biopolymeren (wie z.B. Chitosan) und anionischen Monomeren, wie beispielsweise (Meth)Acrylaten oder Alginaten.

### Verkapselungsverfahren

Bei den Kapseln handelt es sich im Allgemeinen um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Halicrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind aus dem Stand der Technik hinlänglich bekannt [WO 01/01926, WO 01/01927, WO 01/01928, WO 01/01929]. Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

### GEWERBLICHE ANWENDBARKEIT

Weitere Gegenstände der vorliegenden Erfindung betreffen zum einen
- ein Verfahren zur Maskierung des unangenehmen Geschmacks von NSAID-Wirkstoffen sowie Formulierungen, die diese Wirkstoffe enthalten, sowie
- zum anderen ein Verfahren zur Linderung und/oder Reduzierung des Brennens von NSAID-Wirkstoffen sowie Formulierungen, die diese Wirkstoffe enthalten, im Hals und auf der Zunge sowie zur Steigerung der Mundfeuchtigkeit,
bei dem man die Wirkstoffe zusammen mit Dehydroabietinsäure oder einen Extrakt, der Dehydroabietinsäure enthält, formuliert und dann oral verabreicht.

Schließlich umfasst die Erfindung auch die Verwendung von Dehydroabietinsäure oder Extrakten, die Dehydroabietinsäure enthalten zur
(i) Maskierung des bitteren und/oder metallischen Geschmacks von NSAID-Wirkstoffen und gegebenenfalls weiteren Formulierungsbestandteilen;
(ii) Linderung oder Reduzierung des Brennens von NSAID-Wirkstoffen sowie Formulierungen, die diese Wirkstoffe enthalten, im Hals und auf der Zunge;
(iii) Steigerung der Mundfeuchtigkeit;
(iv) Verstärkung der Aromen und der Süße von Formulierungen, die NSAID-Wirkstoffe enthalten;
(v) Verbesserung der Schleimhautverträglichkeit bei der oralen Aufnahme von Formulierungen, die NSAID-Wirkstoffe enthalten.

Soweit vorstehend bereits auf bevorzugte Ausführungsform, speziell auf besonders vorteilhafte NSAID, NSAID-Formulierungen, Hilfsstoffe, Mengen und Mengenverhältnisse eingegangen worden ist, so gelten diese Angaben bezogen auf die beanspruchten Verfahren sowie die Verwendung mutatis-mutandis, ohne dass es weiterer Erwähnung oder Wiederholung bedarf.

Im Folgenden wird die Erfindung an Hand einer Reihe von Ausführungsbeispielen illustriert, ohne sie darauf jedoch einzuschränken.

### BEISPIELE

### HERSTELLBEISPIELE

**BEISPIEL H1**

### Reinigung von Dehydroabietinsäure

2g handelsübliche Dehydroabietinsäure (CAS 1740-19-8, bezogen von Interchim, 211 bis AVENUE KENNEDY, BP 1140, 03103 MONTLUCON CEDEX, Frankreich) mit einem Gehalt von ca. 85% wurde durch präparative HPLC-Chromatographie wie folgt gereinigt.

| | |
|---|---|
| Trennungsnummer: | H-2074-B |
| Stationäre Phase: | LichrospherSelect B, 10 µm, 250 x 50 mm |
| Mobile Phase A: | 5mMol Ammoniumformiatpuffer, mit Ameisensäure auf pH 3,0 eingestellt |
| Mobile Phase B: | Methanol -Acetonitril 1:1 (v/v) mit 5 mMol Ammoniumformiat |
| Gradient: | 62% auf 81% B in 57 min |
| Flussrate: | 80 ml/min |
| Detektion: | ELSD |

Produkthaltige Fraktionen wurden vereinigt, das Lösemittel im Vakuum abgedampft und die isolierte Dehydroabietinsäure über H-NMR-Spektroskopie und LCMS analytisch charakterisiert. Die Identität der isolierten Dehydroabietinsäure wird durch das NMR und die Molmasse bestätigt, die Reinheit lag bei > 98%.

Das LCMS-Verfahren zur analytischen Charakterisierung der isolierten Substanz ist in der nachfolgenden **Tabelle H1** zusammengefasst:

**Tabelle H1**

| LCMS Verfahren | | | |
|---|---|---|---|
| **HPLC System** | PE Series 200 | | |
| **MS System** | Applied Biosystems API 150 | | |
| **Data System** | Analyst 1.3 | | |
| **Stationäre Phase** | Merck Select B 250x4 mm, 5 µm | | |
| **Flussrate** | 1 ml/min | | |
| **Detektion** | (+/(-)-ESI, Fast-Switching-Mode | | |
| | ELSD (Sedex 75) | | |
| | UV (Merck, 254 nm) | | |
| **Probenkonzentration** | 10 mg/ml in DMSO | | |
| **Injektionsvolumen** | 30 µl | | |
| **Mobile Phase:** | A: 5 mM Ammoniumformiat and 0.1 % Ameisensäure | | |
| | B: Acetonitril/methanol = 1:1, 5 mM Ammoniumformiat und 0.1 % Ameisensäure (pH 3) | | |
| **Gradient** | Time [min] | % A | % B |
| | 00.0 | 85 | 15 |
| | 30.0 | 0 | 100 |
| | 35.0 | 0 | 100 |

### BEISPIEL H2

### Herstellung eines dehydroabietinsäurehaltigen Extraktes

10g Handelsübliches Colophonium-Harz (bezogen von der Alfred Galke GmbH, Am Bahnhof 1, 37539 Bad Grund, Bestellnummer 36004) wird mit einem Gemisch aus MTB-Ether und Methanol (1:1 v/v) extrahiert und das Lösemittel im Vakuum entfernt. Der Extrakt enthält ca. 25% Dehydroabietinsäure.

### ANWENDUNGSTECHNISCHE BEISPIELE

In den nachfolgenden **Tabellen A und B** sind Inhaltsstoffe der getesteten Formulierungen (ohne den jeweiligen Wirkstoff laut Beispielen) aufgelistet:

**Tabelle A**

| **Inhaltsstoffe I** | | | |
|---|---|---|---|
| **Konservierungsmittel** | **Süßungsmittel** | **Aroma** | **Hilfsmittel** |
| Methylparahydroxybenzoate (E218, 219) | Sorbitol (E420) | Strawberry | Glycerol acetylated monoglycerides propylene glycol |
| | Maltitol (E965) | Orange | |
| Propylparahydroxybenzoate (E216, 217) | Acesulfam K | | |
| | Saccharin | | Mg-Stearate |
| Methylparaben | Sucrose | | Na-Laurylsulfat |
| Propylparaben | | | Stearinsäure |
| Na-Metabisulphite (E223) | | | French chalk (Silikat) |
| Domiphen bromide | | | Povidone |
| Sodium benzoate | | | polysorbate 80 |
| | | | Na-Starch glycollate Type A croscarmellose sodium colloidal silicon dioxide |

**Tabelle B**

| **Inhaltsstoffe II** | |
|---|---|
| **Füllstoffe/Verdickungsmittel** | **Farbstoffe/Beschichtung** |
| Mikrokristalline Zellulose | Kolloidales Silikon-Dioxid (E171) |
| Maisstärke | Opaspray White M-1-7111B (contains Hypromellose, Titanium Dioxide E171) |
| Xanthan gum | |
| Dispersible Cellulose | Blanc Ink (contains shellac, Iron oxide black E172) |
| Hypromellose | pharmaceutical ink |
| Pregelatinized starch | pharmaceutical glaze |
| | synthetic iron oxide |
| | white wax |

### BEISPIEL 1

### Geschmacksmodulierung von Paracetamol in einer kommerziell erhältlichen Formulierung

Die sensorische Beurteilung der Proben wurde von einem Team aus fünf erfahrenen Prüfern durchgeführt. Die Einzelheiten sind der folgenden **Tabelle 1** zu entnehmen:

**Tabelle 1**

| Verkostung von Calpol^{®}-Lösung | |
|---|---|
| **Testmethode** | Deskriptive und diskriminierende Prüfung, "sip and spit"-Methode, Proben verblindet und randomisiert |
| **Materialien** | Gereinigte Dehydroabietinsäure, Reinheit > 98% |
| | Calpol^{®}: zuckerfreie Suspension mit Erdbeergeschmack, gesüßt mit Acesulfam K und Na-Saccharin |
| **Panelist** | 5 erfahrene Testpersonen |
| **Probenvorbereitung** | (A) Dehydroabietinsäure gelöst in Ethanol |
| | (B) Calpol^{®} Suspension in Wasser, Endkonzentration Paracetamol betrug 12,5 mg/ml |
| | Probe: (A)+(B), final 25 µM Dehydroabietinsäure, 0,5 % Ethanol |
| | Vergleichsprobe: (B) incl. 0,5 % Ethanol |
| **Bewertung der Probe** | - sehr viel weniger bitter |
| Calpol^{®} Suspension in Wasser plus 25 µM Dehydroabietinsäure | - metallischer Geschmack reduziert |

### BEISPIEL 2

### Geschmacksmodulierung von Paracetamol in einer Maltitol-Formulierung

Die sensorische Beurteilung der Proben wurde von einem Team aus fünf erfahrenen Prüfern durchgeführt. Die Einzelheiten sind der folgenden **Tabelle 2** zu entnehmen:

**Tabelle 2**

| Verkostung einer Maltitol-Paracetamol-Lösung | |
|---|---|
| **Testmethode** | Deskriptive und diskriminierende Prüfung, "sip and spit"-Methode, Proben verblindet und randomisiert |
| **Testprobe** | Gereinigte Dehydroabietinsäure, Reinheit > 98% Suspension von Paracetamol gelöst in Maltitolsirup |
| **Panelist** | 5 erfahrene Testpersonen |
| **Probenvorbereitung** | (A) Dehydroabietinsäure gelöst in Ethanol |
| | (B) Paracetamol-Maltitol-Sirup gelöst in Wasser, Endkonzentration Paracetamol betrug 12,5 mg/ml |
| | Probe: (A)+(B), final 25 µM Dehydroabietinsäure, 0,5 % Ethanol |
| | Vergleichsprobe: (B) incl. 0,5 % Ethanol |
| **Bewertung der Probe** | - Bitterkeit reduziert und verzögert |
| Paracetamol-Maltitol-Sirup in Wasser plus 25 µM Dehydroabietinsäure | - kein langanhaltender unangenehmer/ metallischer Geschmack |
| | - besserer Geschmack zu Beginn und angenehmerer Nachgeschmack |

### BEISPIEL 3

### Geschmacksmodulierung von Ibuprofen in Tablettenform mit Lysin (Nurofen^{®})

Die sensorische Beurteilung der Proben wurde von einem Team aus fünf erfahrenen Prüfern durchgeführt. Die Einzelheiten sind der folgenden **Tabelle 3** zu entnehmen:

**Tabelle 3**

| Verkostung einer Nurofen^{®}-Lösung | |
|---|---|
| **Testmethode** | Deskriptive und diskriminierende Prüfung, "sip and spit"-Methode, Proben verblindet und randomisiert |
| **Testprobe** | Gereinigte Dehydroabietinsäure, Reinheit > 98% Suspension von Nurofen^{®} Tabletten in Wasser |
| **Panelist** | 5 erfahrene Testpersonen |
| **Probenvorbereitung** | (A) Dehydroabietinsäure gelöst in Ethanol |
| | (B) Nurofen^{®} Tabletten gelöst in Wasser, Endkonzentration Ibuprofen betrug 5 mg/ml |
| | Probe: (A)+(B), final 25 µM Dehydroabietinsäure, 0,5 % Ethanol |
| | Vergleichsprobe: Nurofen^{®} Tabletten gelöst in Wasser mit 0,5 % Ethanol |
| **Bewertung der Probe** | - Bitterkeit reduziert |
| Nurofen^{®} Tabletten gelöst in Wasser plus 25 µM Dehydroabietinsäure | - keine Veränderung des verzögerten Brennens im Hals |

### BEISPIEL 4

### Geschmacksmodulierung von Ibuprofen in Suspension (Nurofen^{®})

Die sensorische Beurteilung der Proben wurde von einem Team aus fünf erfahrenen Prüfern durchgeführt. Die Einzelheiten sind der folgenden **Tabelle 4** zu entnehmen:

**Tabelle 4**

| Verkostung einer Nurofen^{®}-Suspensions-Lösung | |
|---|---|
| **Testmethode** | Deskriptive und diskriminierende Prüfung, "sip and spit"-Methode, Proben verblindet und randomisiert |
| **Testprobe** | Gereinigte Dehydroabietinsäure, Reinheit > 98% |
| | Nurofen^{®}: zuckerfreie Suspension mit Erdbeergeschmack, gesüßt mit Na-Saccharin |
| **Panelist** | 5 erfahrene Testpersonen |
| **Probenvorbereitung** | (A) Dehydroabietinsäure gelöst in Ethanol |
| | (B) Nurofen^{®} Suspension gelöst in Wasser, Endkonzentration Ibuprofen betrug 5 mg/ml |
| | Probe: (A)+(B), final 25 µM Dehydroabietinsäure, 0,5 % Ethanol |
| | Vergleichsprobe: Nurofen^{®} Suspension gelöst in Wasser mit 0,5 % Ethanol |
| **Bewertung der Probe** | Maskierung des typischen Eigengeschmacks von Ibuprofen. |
| Nurofen^{®} Suspension gelöst in Wasser plus 25 µM Dehydroabietinsäure | |

### BEISPIEL 5

### Geschmacksmodulierung von Ibuprofen in Suspension (Asda)

Die sensorische Beurteilung der Proben wurde von einem Team aus fünf erfahrenen Prüfern durchgeführt. Die Einzelheiten sind der folgenden **Tabelle 5** zu entnehmen:

**Tabelle 5**

| Verkostung einer Ibuprofen-Suspensions-Lösung | |
|---|---|
| **Testmethode** | Deskriptive und diskriminierende Prüfung, "sip and spit"-Methode, Proben verblindet und randomisiert |
| **Testprobe** | Gereinigte Dehydroabietinsäure, Reinheit > 98% |
| | Ibuprofen Suspension: Suspension mit Orangengeschmack, ohne Süßstoffe |
| **Panelist** | 5 erfahrene Testpersonen |
| **Probenvorbereitung** | (A) Dehydroabietinsäure gelöst in Ethanol |
| | (B) Ibuprofen-Suspension gelöst in Wasser, Endkonzentration Ibuprofen betrug 5 mg/ml |
| | Probe: (A)+(B), final 25 µM Dehydroabietinsäure, 0,5 % Ethanol |
| | Vergleichsprobe: Ibuprofen-Suspension gelöst in Wasser mit 0,5 % Ethanol |
| **Bewertung der Probe** | - weniger bitter |
| Ibuprofen- Suspension gelöst in Wasser plus 25 µM Dehydroabietinsäure | - süßer |
| | - Verstärkung des Orangenaromas |
| | - angenehmer im Geschmack |

### BEISPIEL 6

### Geschmacksmodulierung von Ibuprofen in einer Maltitol-Formulierung

Die sensorische Beurteilung der Proben wurde von einem Team aus fünf erfahrenen Prüfern durchgeführt. Die Einzelheiten sind der folgenden **Tabelle 6** zu entnehmen:

**Tabelle 6**

| Verkostung einer Maltitol- Ibuprofen -Lösung | |
|---|---|
| **Testmethode** | Deskriptive und diskriminierende Prüfung, "sip and spit"-Methode, Proben verblindet und randomisiert |
| **Testprobe** | Gereinigte Dehydroabietinsäure, Reinheit > 98% |
| | Suspension von Ibuprofen gelöst in Maltitolsirup |
| **Panelist** | 5 erfahrene Testpersonen |
| **Probenvorbereitung** | (A) Dehydroabietinsäure gelöst in Ethanol |
| | (B) Ibuprofen-Maltitol-Sirup gelöst in Wasser, Endkonzentration Paracetamol betrug 5 mg/ml |
| | Probe: (A)+(B), final 25 µM Dehydroabietinsäure, 0,5 % Ethanol |
| | Vergleichsprobe: Ibuprofen -Sirup in Wasser mit 0,5 % Ethanol |

| | |
|---|---|
| **Bewertung der Probe** - weniger bitter Ibuprofen -Maltitol-Sirup - weniger unangenehm im Nachgeschmack in Wasser plus 25 µM - reduzierte Mundtrockenheit Dehydroabietinsäure - leicht süß - Linderung des Brennens auf der Zunge/im Hals | |

### FORMULIERUNGSBEISPIELE

### Brausetablette

500 mg Paracetamol
5 mg Dehydroabietinsäure
200 mg Ascorbinsäure
50 mg Natriumcyclamat
8 mg Natriumsaccharinat
327 mg Sorbitol
30 mg Fruchtaroma
20 mg Propylenglykol
1.160mg Citronensäure
1.000 mg Natriumcarbonat
Gewicht der Brausetablette 3.3g, entsprechend 15% Wirkstoffgehalt.

### Retardtablette

500 mg Acetylsalicylsäure
1 mg Dehydroabietinsäure
100 mg Maisstärke oder vorverkleisterte Maisstärke
Tablettengewicht 600 mg, entsprechend 83% Wirkstoffgehalt.

### Gelatine- oder Alginatkapsel

Ibuprofen und Dehydroabietinsäure werden zusammen mit Colestipol granuliert und in eine Gelatinekapsel gefüllt (333mg Gewicht bei 200mg Wirkstoff, entsprechend 60% Wirkstoffgehalt).

### Tablette

256,25 kg Ibuprofen-Natrium-Dihydrat, 0,2 kg Dehydroabietinsäure, 25kg Povidone K25 und 46,75kg Natriumhydrogencarbonat werden zu Tabletten à 331 mg verpresst. Der Wirkstoffgehalt beträgt ca. 78%

## Patentansprüche

1. Zubereitung, enthaltend
(a) mindestens einen NSAID-Wirkstoff und
(b) Dehydroabietinsäure oder einen Extrakt, der Dehydroabietinsäure enthält als Arzneimittel.

2. Zubereitung, enthaltend
(a) mindestens einen NSAID-Wirkstoff und
(b) Dehydroabietinsäure oder einen Extrakt, der Dehydroabietinsäure enthält zur Verwendung als Arzneimittel zur Behandlung und/oder Prophylaxe von Schmerzzuständen, Entzündungszuständen und rheumatischen Erkrankungen.

3. Zubereitungen nach den Ansprüchen 1 oder 2, **dadurch gekennzeichnet, dass** sie NSAID-Wirkstoffe enthalten, die ausgewählt sind aus der Gruppe, die gebildet wird von Acetylsalicylsäurederivaten, Arylpropionsäurederivaten, Arylessigsäurederivaten, Indolessigsäurederivaten, Anthranilsäurederivaten, Oxicamen sowie deren Mischungen.

4. Zubereitungen nach Anspruch 3, **dadurch gekennzeichnet, dass** sie NSAID-Wirkstoffe enthalten, die ausgewählt sind aus der Gruppe, die gebildet wird von Acetylsalicylsäure (Aspirin), Ibuprofen, Flurbiprofen, Naproxen, Ketoprofen, Tiaprofensäure, Diclofenac, Indometacin, Flufenaminsäure, Mefenaminsäure, Piroxicam, Tenoxicam, Meloxicam, para-Acetylaminophenol (Paracetamol) sowie deren Mischungen.

5. Zubereitungen nach mindestens einem der Ansprüche 1 bis 4, **dadurch gekennzeichnet, dass** sie als Komponente (c) außerdem mindestens einen Süßstoff enthalten.

6. Zubereitungen nach Anspruch 5, **dadurch gekennzeichnet, dass** sie Süßstoffe enthalten, die ausgewählt sind aus der Gruppe, die gebildet wird von Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.), Agaven (Agavendicksaft, Fruchtkonzentrate (z.B. auf Basis von Äpfeln oder Birnen), Erythritol, Threitol, Arabitol, Ribotol, Xylitol, Sorbitol, Mannitol, Dulcitol, Lactitol), Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Glycin, D-Leucin, D-Threonin, D-Asparagin, D-Phenylalanin, D-Tryptophan, L-Prolin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie Extrakten der Pflanzen der Gattung *Rubus* sowie deren Gemischen.

7. Zubereitungen nach mindestens einem der Ansprüche 1 bis 6, **dadurch gekennzeichnet, dass** sie als Komponente (d) außerdem mindestens einen Träger enthalten.

8. Zubereitungen nach Anspruch 7, **dadurch gekennzeichnet, dass** sie entweder feste Träger, die ausgewählt sind aus der Gruppe, die gebildet wird von Dextrinen, Cellulosen, insbesondere Mikrocellulosen, Stärken und modifizierten Stärken, Xanthan Gum sowie deren Mischungen oder flüssige Träger enthalten, bei denen es sich um Wasser, Ethanol, Glycerin oder Ethylenglykol oder deren Mischungen handeln kann.

9. Zubereitungen nach mindestens einem der Ansprüche 1 bis 8, **dadurch gekennzeichnet, dass** sie als feste oder flüssige Formulierungen vorliegen.

10. Zubereitungen nach Anspruch 9, **dadurch gekennzeichnet, dass** sie als Tabletten, Lutschbonbons, Kaugummis oder Kapseln vorliegen.

11. Zubereitungen nach Anspruch 9, **dadurch gekennzeichnet, dass** sie als Saft oder Spray vorliegen.

12. Zubereitungen nach mindestens einem der Ansprüche 1 bis 11, **dadurch gekennzeichnet, dass** sie
(a) etwa 0,01 bis etwa 90 Gew.-% NSAID-Wirkstoffe
(b) etwa 0,001 bis etwa 5 Gew.-% Dehydroabietinsäure oder einen entsprechenden Extrakt,
(c) 0 bis etwa 15 Gew.-% Süßstoffe
(d) 0 bis etwa 95 Gew.-% Träger
mit der Maßgabe enthalten, dass sich die Mengenangaben gegebenenfalls mit weiteren Hilfs- und Zusatzstoffen zu 100 Gew.-% ergänzen.

13. Verfahren zur Maskierung des unangenehmen Geschmacks von NSAID-Wirkstoffen sowie Formulierungen, die diese Wirkstoffe enthalten, bei dem man die Wirkstoffe zusammen mit Dehydroabietinsäure oder einen Extrakt, der Dehydroabietinsäure enthält, formuliert und dann oral verabreicht.

14. Verfahren zur Linderung und/oder Reduzierung des Brennens von NSAID-Wirkstoffen sowie Formulierungen, die diese Wirkstoffe enthalten, im Hals und auf der Zunge sowie zur Steigerung der Mundfeuchtigkeit, bei dem man die Wirkstoffe zusammen mit Dehydroabietinsäure oder einen Extrakt, der Dehydroabietinsäure enthält, formuliert und dann oral verabreicht.

15. Verwendung von Dehydroabietinsäure oder Extrakten, die Dehydroabietinsäure enthalten zur
(i) Maskierung des bitteren und/oder metallischen Geschmacks von NSAID-Wirkstoffen und gegebenenfalls weiteren Formulierungsbestandteilen;
(ii) Linderung oder Reduzierung des Brennens von NSAID-Wirkstoffen sowie Formulierungen, die diese Wirkstoffe enthalten, im Hals und auf der Zunge;
(iii) Steigerung der Mundfeuchtigkeit;
(iv) Verstärkung der Aromen und der Süße von Formulierungen, die NSAID-Wirkstoffe enthalten; sowie
(v) Verbesserung der Schleimhautverträglichkeit bei der oralen Aufnahme von Formulierungen, die NSAID-Wirkstoffe enthalten.
